# EUROPEAN PATENT SPECIFICATION

(11) **EP 0 691 853 B1**
(45) Date of publication and mention of the grant of the patent: **05.06.2002**
(21) Application number: 94912336.8
(22) Date of filing: 29.03.1994
(51) Int. Cl.: A61K 48/00, A61K 31/70, A61K 31/74, A61K 31/795, C07H 21/04

(54) **ANTISENSE OLIGOS WHICH INTERFERE WITH mRNA CAP ACTIVITY AND INHIBIT TRANSLATION**
ANTISENSE OLIGONNUKLEOTIDE DIE MIT DER mRNA CAP AKTIVITÄT INTERFERIEREN UND DIE TRANSLATION INHIBIEREN
OLIGONUCLEOTIDES ANTISENS INTERFERANT AVEC L'ACTIVITE DE COIFFE DE L'ARNm ET INHIBANT LA TRADUCTION

(30) Priority: 01.04.1993 US 45268
(43) Date of publication of application: 17.01.1996
(73) Proprietor: ISIS PHARMACEUTICALS, INC., Carlsbad, CA 92008 (US)
(72) Inventor: BAKER, Brenda F., Encinitas, CA 92024 (US)
(74) Representative: Hallybone, Huw George
(86) International application number: US9403401
(87) International publication number: WO9422488

(56) References cited:
- WO-A-91/17755
- BAKER B F ET AL: "Modulation of eucaryotic initiation factor-4E binding to 5'- capped oligoribonucleotides by modified anti - sense oligonucleotides." JOURNAL OF BIOLOGICAL CHEMISTRY, (1992 JUN 5) 267 (16) 11495-9., XP002060266
- GOODCHILD, J. ET AL.: "Inhibition of rabbit beta-globin synthesis by complementary oligonucleotides: identification of mRNA sites sensitive to inihibition" ARCHIVES OF BIOCHEMISTRY AND BIOPHYSICS, vol. 263, June 1988, page 401-9 XP002060267
- HELENE, C. & TOULME, J.-J.: "SPECIFIC REGULATION OF GENE EXPRESSION BY ANTISENSE, SENSE AND ANTIGENE NUCLEIC ACIDS" BIOCHIMICA ET BIOPHYSICA ACTA, vol. 1049, 1990, pages 99-125, XP000570355
- Biomedica et Biochimica Acta, Volume 48, Number 1, issued 1989, WESTERMANN et al., "Inhibition of Expression of SV40 Virus Large T-Antigen by Antisense Oligodeoxyribonucleotides", pages 85-93, especially pages 90-91.
- Cell, Volume 63, issued 05 October 1990, J. HAMM et al., "Monomethylated Cap Structures Facilitate RNA Export from the Nucleus", pages 109-118, especially page 112.
- Molecular Biology of Medicine, Volume 5, issued 1988, J. ROSS, "Messenger RNA Turnover in Eukaryotic Cells", pages 1-14, especially page 7.
- Annual Review of Biochemistry, Volume 54, issued 1985, K. MOLDAVE, "Eukaryotic Protein Synthesis", pages 1109-1149, especially pages 1121-1125.
- Chemical Reviews, Volume 90, No. 4, issued June 1990, E. UHLMANN et al., "Antisense Oligonucleotides: A New Therapeutic Principle", pages 543-584, especially pages 546-558.
- Journal of the American Chemical Society, Volume 113, issued 1991, A. MODAK et al., "Toward Chemical Ribonucleases. 2. Synthesis and Characterization of Nucleoside-Bipyridine Conjugates. Hydrolytic Cleavage of RNA by their Copper(II) Complexes", pages 283-291, especially pages 287-288.
- Gene, Volume 61, issued 1987, P. VERSPIEREN et al., "An Acridine-Linked Oligodeoxynucleotide Targeted to the Common 5' End of Trypanosome mRNAs Kills Cultured Parasites", pages 307-315, especially pages 309-311.
- Journal of the American Chemical Society, Volume 111, J. CHIN et al., "Co(III) Complex Promoted Hydrolysis of Phosphate Diesters: Comparison in Reactivity of Rigid cis-Diaquotetraazacobalt(III) Complexes", pages 186-190, especially page 188.
- D. APIRION, "Processing of RNA", published 1984 by CRC Press, Inc. (Boca Raton, FL), pages 119-132, see pages 121-124.

## Description

### FIELD OF THE INVENTION

This invention is directed to the use of antisense oligonucleotides for achieving therapeutic treatment of diseases and for regulating gene expression in biological experimental systems. The invention generally relates to the field of antisense oligonucleotide compounds in conjunction with the field of gene expression, especially protein expression, which is dependent upon the 5' cap of eucaryotic and viral RNAs.

### BACKGROUND OF THE INVENTION

Classical therapeutics has generally focused upon interactions with proteins in efforts to moderate their disease-causing or disease-potentiating functions. Such therapeutic approaches have many limitations. Recently, antisense oligonucleotide therapeutic and diagnostic approaches have been proposed.

Antisense methodology is the complementary hybridization of relatively short oligonucleotides to single-stranded mRNA or single-stranded DNA, or even double-stranded DNA, such that the normal, essential functions of these intracellular nucleic acids are disrupted. Hybridization is the sequence specific hydrogen bonding of oligonucleotides to RNA or single stranded DNA according to the rules of Watson-Crick base pairing. Such base pairs are said to be complementary to one another.

The events which disrupt nucleic acid function are discussed by Cohen in *Oligonucleotides: Antisense Inhibitors of Gene Expression,* CRC Press, Boca Raton FL, (1989) who proposes two possible types of terminating events. The first, hybridization arrest, denotes a terminating event in which the oligonucleotide inhibitor binds to the target nucleic acid and thus prevents, by simple steric hindrance, the binding of essential proteins, such as ribosomes or capping enzymes, to the nucleic acid. Methyl phosphonate oligonucleotides; P. S. Miller & P.O.P. Ts'O, *Anti-Cancer Drug Design,* Vol. 2, pp. 117-128 (1987); and α-anomer oligonucleotides, Cohen J.S. ed., *Oligonucleotides: Antisense Inhibitors of Gene Expression*, CRC Press, Boca Raton FL (1989) are two of the most extensively studied antisense agents which are thought to disrupt nucleic acid function by hybridization arrest.

A second type of terminating event for antisense oligonucleotides involves enzymatic cleavage of the targeted RNA by intracellular RNase H. The oligonucleotide, which must be of the deoxyribo type, hybridizes with the targeted RNA and this duplex activates the RNase H enzyme to cleave the RNA strand, thus destroying the normal function of the RNA. Phosphorothioate oligonucleotides are a prominent example of an antisense agent which operates by this type of terminating event.

Considerable research is being directed to the application of oligonucleotides as antisense agents for therapeutic purposes. Applications of oligonucleotides as diagnostics, research reagents, and potential therapeutic agents require that the oligonucleotides be synthesized in large quantities, be transported across cell membranes or taken up by cells, appropriately hybridize to targeted RNA or DNA, and subsequently terminate or disrupt nucleic acid function. These critical functions depend on the initial stability of oligonucleotides towards nuclease degradation. Other methodologies of action for oligonucleotide therapeutics and diagnostics have been sought in recent years and therapeutic and diagnostic compounds and compositions to exploit such methodologies are greatly desired. The present invention provides such new compounds and compositions together with methodologies for the use of antisense oligonucleotides in the diagnosis and treatment of disease in animals.

Most antisense approaches utilize antisense oligonucleotides targeted to the AUG, or, less often, the 5'-untranslated region, 3'-untranslated region, coding or other regions of mRNA. It is also known to use antisense oligonucleotides which target the 5' cap of mRNA to interfere with mRNA function, as disclosed in PCT publication WO 91/17755 (Baker), which is assigned to the same assignee as the present invention. In the present invention, antisense oligonucleotides are used to prevent capping of the 5' end of mRNA in order to achieve the desired effect of interfering with mRNA function. Interference with mRNA function by preventing capping has been heretofore unknown.

Thus, there has been a long-felt need for oligonucleotides which interfere with mRNA function, are effective for therapeutic use and are possessed of no or few side effects. The present invention addresses this need.

### OBJECTS OF THE INVENTION

It is a principal object of this invention to provide therapeutic methods and materials for the treatment of diseases through modulation of the activity of mRNA.

It is a further object of this invention to provide oligonucleotides for use in therapeutics.

A further object of this invention is to provide such oligonucleotides which are less likely to cause undesired or toxic side reactions or effects.

Another object of this invention is to provide oligonucleotides that inhibit the maturation, stabilization, and/or translation of a selected mRNA.

Yet another object of this invention is to provide oligonucleotides that interfere with the capping of nascent mRNA.

A further object of this invention is to provide oligonucleotides that sterically or chemically interfere with the capping of nascent mRNA.

These and other objects will become apparent to persons of ordinary skill in the art from a review of the present specification and attendant claims.

### SUMMARY OF THE INVENTION

This invention provides the use of oligonucleotide compounds for inhibiting the capping of a nascent mRNA. The term "nascent mRNA" is commonly used to refer to mRNA molecules which are in the process of being transcribed (synthesized). The oligonucleotides are designed to be specifically hybridizable with the nascent mRNA to be inhibited and to interfere with the action or effect of capping enzyme upon the nascent mRNA. In preferred embodiments, the oligonucleotide compounds comprise modifications or functionalities which interfere with the capping reaction. The effect of the contacting of compounds of the invention with the targeted nascent mRNA is to impede or prevent the 5' capping of the nascent mRNA by enzymes such as guanylyl transferase such that it does not fully mature into mRNA. The incompletely matured mRNA either fails to be translated into protein altogether, or is translated to a diminished or impaired degree.

Since capping is generally a function of the 5' end of nascent mRNA, the specific hybridization desired is generally to a 5' portion of the nascent mRNA. The nascent mRNAs to be inhibited are preferrably from about 20 to about 80 nucleotides in length. The compounds of the invention may operate by several mechanisms including steric interference with the 5' end of the nascent mRNA such that capping enzymes are prevented from performing their function. In a preferred embodiment, one or more nucleotides of the oligonucleotide of the invention "hang over" or "extend beyond" the 5' terminus of the nascent mRNA. By this is meant that the 3' portion of the oligonucleotide compound has nucleotide units in excess of those needed to hybridize with the 5' portion of the nascent mRNA.

Alternatively, the compounds, generally acting through their modifications or functionalities, may be such as to chemically modify the nascent mRNA in a manner inconsistent with its maturation. Such modification is preferably through the dephosphorylation of the 5' triphosphate or diphosphate of the nascent mRNA. The result of such a modification is generally a 5' monophosphate or hydroxyl group.

The methods of the invention inhibit capping of a nascent mRNA by interfering with the capping of a nascent mRNA, generally selected as being one which leads to a mature mRNA which is essential to the development or maintenance of a disease state. Generally the mRNA (and hence the nascent mRNA) selected is cell-, tissue- or disease-specific. The nascent mRNA is contacted with an oligonucleotide compound of the invention either directly, such as would be the case in certain diagnostic applications, or in cells, tissues or bodily fluids containing the nascent mRNA. The compound is present in an amount effective to diminish the mRNA activity or function. A preferred function to be interfered with is export of mRNA from cell nuclei. Another is splicing of the mRNA, and yet another is translation of the mRNA into protein.

### DETAILED DESCRIPTION

It has been recognized that the majority of eucaryotic and viral small nuclear RNAs and messenger RNAs have a unique chemical structure at their 5' terminus which is required in varying degrees for their maturation, stability, and efficiency of translation. The general structural features are given in *J. Mol. Biol.* 99 519-547 (1975). The cap is a guanosine residue which is methylated at the nitrogen 7 position. It is joined to the penultimate 5' base of the RNA via a triphosphate linkage between the 5' hydroxyl groups of each residue. The 2' hydroxyls of the 5' terminal base(s) are methylated.

The cap structure is added to the 5' end of nascent mRNA transcripts during transcription in the nucleus. The term "nascent mRNA" is commonly used to refer to mRNA molecules which are in the process of being transcribed (synthesized). Capping occurs when the nascent mRNA, which emerges from the transcription machinery with the 5' end first, is from about 20 to 80 nucleotides in length. Coppola, J.A., Field, A.S., and Luse, D.S. (1983) *Proc. Natl. Acad. Sci.* 80:1251-1255. The cap site corresponds to the transcription initiation site of the mRNA. Capping is believed to occur in four steps: (1) hydrolysis of the 5'-terminal triphosphate of the nascent mRNA transcript to a diphosphate; (2) covalent attachment of a guanylate moiety to the 5' terminus via a 5'-to-5' triphosphate linkage; (3) methylation of the new guanine cap at the N7 position; and (4) methylation of the 2'-hydroxyl group of the first encoded nucleotide. These reactions are believed to be carried out by multifunctional protein complexes. At least three enzymatic activities are involved in the capping process: (1) an RNA triphosphate activity removes the 5'-terminal phosphate from the 5'-triphosphate of the nascent mRNA, (2) a guanylyltransferase activity transfers the guanine residue to the 5'-diphosphate of the nascent mRNA and, (3) a methyl transferase activity which methylates the guanine residue.

Once transcribed, the primary transcript from eucaryotic and certain viral genes must be processed due to the presence of intervening sequences within the coded regions of the transcript. The 5' cap has been shown to be necessary for processing of the primary transcripts to mature RNA molecules, specifically in the splicing reactions during which removal of the first intron occurs. See *Cell* 38 731-736 (1984). Absence of a 5' cap results in rapid degradation of the RNA in the nucleus and the cytoplasm as described in *Mol. Biol. Med*. 5 1-14 (1988) and *Cell* 32 681-694 (1983). Without the 5' cap the RNA is presumably accessible to 5' exonucleases. The 5' cap also plays an important role in transport of mRNA out of the nucleus. Hamm, J. and Mattaj, I.W. (1990) *Cell* 63:109. Finally, the majority of eucaryotic and many viral mRNAs studied to date require the 5' cap for the initiation of protein translation; See *Cell* 9 645-653 (1976); *Federation of Experimental Biologists Society Letter* 96 1-11(**1978**); and *Prog. Nuc. Acid Res.* 35 173-207 (1988). There are also specific cap binding proteins which are components of the machinery required for the initiation of translation of a protein. See *Cell* 40 223-24 (1985); and *Prog. Nuc. Acid Res.* 35 173-207 (1988).

Based on the current understanding of the properties and function of the 5' cap structure of messenger RNA it is now believed that chemical or steric interference which prevents capping of nascent mRNA transcripts will preclude the transcripts from any pertinent processes, such as the production of an undesired protein. In general, the compositions necessary for modulating the activity of an RNA transcript in accordance with this invention may be regarded as oligonucleotides which sterically block or chemically modify the targeted nascent mRNA transcript to make the mRNA inaccessible to, or unrecognizable by, a capping enzyme, preferably an RNA triphosphatase or guanylyltransferase. The resulting uncapped transcript is unable to operate in one or more of its normal processes.

The oligonucleotides that are used in accordance with this invention specifically hybridize with the selected nascent transcript in sufficient proximity to the 5' end to block or modify the site for 5' capping. The oligonucleotides of the present invention preferably have about 5 to about 50 nucleotide units. It is more preferred that such functionalities have from about 10 to 25 nucleotide units. As will be appreciated, a nucleotide unit is a base-sugar combination suitably bound to an adjacent nucleotide unit through phosphodiester or other bonds.

In the context of this invention, the term "oligonucleotide" refers to an oligomer or polymer of ribonucleic acid or deoxyribonucleic acid. This term includes oligomers consisting of naturally occurring bases, sugars and intersugar (backbone) linkages as well as oligomers having non-naturally occurring portions which function similarly. Such modified or substituted oligonucleotides are often preferred over native forms because of properties such as, for example, enhanced cellular uptake and increased stability in the presence of nucleases.

Specific examples of some preferred oligonucleotides envisioned for use in this invention may contain phosphorothioates, phosphotriesters, methyl phosphonates, short chain alkyl or cycloalkyl intersugar linkages or short chain heteroatomic or heterocyclic intersugar linkages. Most preferred are those with CH₂-NH-O-CH₂, CH₂-N(CH₃)-O-CH₂, CH₂-O-N(CH₃)-CH₂, CH₂-N(CH₃)-N(CH₃)-CH₂ and O-N(CH₃)-CH₂-CH₂ backbones (where phosphodiester is O-P-O-CH₂). Also preferred are oligonucleotides haying morpholino backbone structures. Summerton, J.E. and Weller, D.D., U.S. Patent 5,034,506. In other preferred embodiments, such as the protein-nucleic acid (PNA) backbone, the phosphodiester backbone of the oligonucleotide may be replaced with a polyamide backbone, the bases being bound directly or indirectly to the aza nitrogen atoms of the polyamide backbone. P.E. Nielsen, M. Egholm, R.H. Berg, O. Buchardt, *Science* **1991**, 254, 1497. Other preferred oligonucleotides may contain alkyl and halogen-substituted sugar moieties comprising one of the following at the 2' position: OH, SH, SCH₃, F, OCN, O(CH₂)ₙNH₂ or O(CH₂)ₙCH₃ where n is from 1 to about 10; C₁ to C₁₀ lower alkyl, substituted lower alkyl, alkaryl or aralkyl; Cl; Br; CN; CF₃; OCF₃; O-, S-, or N-alkyl; O-, S-, or N-alkenyl; SOCH₃; SO₂CH₃; ONO₂; NO₂; N₃; NH₂; heterocycloalkyl; heterocycloalkaryl; aminoalkylamino; polyalkylamino; substituted silyl; a fluorescein moiety, an RNA cleaving group; a conjugate; a reporter group; an intercalator; a group for improving the pharmacokinetic properties of an oligonucleotide; or a group for improving the pharmacodynamic properties of an oligonucleotide and other substituents having similar properties. In a preferred embodiment, oligonucleotides may contain one or more moieties capable of interfering with capping of the 5' end of a nascent mRNA target, either through steric interference or chemical modification of the nascent mRNA. In one preferred embodiment, the chemical modification is dephosphorylation of the 5'-triphosphate or 5'-diphosphate of the nascent mRNA, yielding a 5'-monophosphate or a hydroxyl group. The interfering moiety may preferrably comprise an amino acid, peptide, nucleotide, polyamine, coordination complex of a metal, metal ion, or a macrocycle. In one preferred embodiment, the interfering portion comprises at least one nucleotide. The nucleotide or nucleotides of the interfering portion may be complementary to the nucleotides of the target mRNA according to the rules of Watson-Crick base pairing or may extend or overlap beyond the 5' end of the nascent mRNA.

Preferred examples of polyamines are triethylamine tetramine, diethylene triamine, and pentethylamine hexamine. Preferred examples of metal ions are transition metals such as cobalt, zinc, and copper; lanthanides such as europium and gadolinium, and divalent cations such as calcium and magnesium. Preferred examples of coordination complexes of metals are copper, zinc, cobalt or lanthanide complexes of bipyridine, orthophenanthroline, 12aneN3, diethylenetriamine pentaacetic acid, or N-[2-mercaptopropionyl]glycine. A preferred example of a heterocycle is the Lehn macrocycle, [24] ane N₆O₂. In a preferred embodiment of the invention, the interfering moiety is attached to the oligonucleotide at the 3' position. Oligonucleotides may also have sugar mimetics such as cyclobutyls or other carbocyclics in place of the pentofuranosyl group. Nucleotide units having nucleosides other than adenosine, cytidine, guanosine, thymidine and uridine may be used, for example inosine.

Chimeric oligonucleotides can also be employed; these molecules contain two or more chemically distinct regions, each comprising at least one nucleotide. These oligonucleotides typically contain a region of modified nucleotides that confer one or more beneficial properties (such as, for example, increased nuclease resistance, increased uptake into cells, increased binding affinity for the RNA target) and an unmodified region that retains the ability to direct RNase H cleavage.

The oligonucleotides used in accordance with this invention may be conveniently and routinely made through the well-known technique of solid phase synthesis. Equipment for such synthesis is sold by several vendors including Applied Biosystems. Any other means for such synthesis may also be employed, however the actual synthesis of the oligonucleotides are well within the talents of the routineer. It is also well known to use similar techniques to prepare other oligonucleotides such as the phosphorothioates and alkylated derivatives.

While the present invention has been described with specificity in accordance with certain of its preferred embodiments, the following examples serve only to illustrate the invention and are not intended to limit the same.

### EXAMPLES

### Example 1

### Oligonucleotide synthesis

Unmodified DNA oligonucleotides are synthesized on an automated DNA synthesizer (Applied Biosystems model 380B) using standard phosphoramidite chemistry with oxidation by iodine. β-cyanoethyldiisopropyl-phosphoramidites are purchased from Applied Biosystems (Foster City, CA). For phosphorothioate oligonucleotides, the standard oxidation bottle is replaced by a 0.2 M solution of 3H-1,2-benzodithiole-3-one 1,1-dioxide in acetonitrile for the stepwise thiation of the phosphite linkages. The thiation cycle wait step is increased to 68 seconds and is followed by the capping step.

2'-O-methyl phosphorothioate oligonucleotides are synthesized using 2'-O-methyl β-cyanoethyldiisopropylphosphoramidites (Chemgenes, Needham MA) and the standard cycle for unmodified oligonucleotides, except the wait step after pulse delivery of tetrazole and base is increased to 360 seconds.

After cleavage from the controlled pore glass column (Applied Biosystems) and deblocking in concentrated ammonium hydroxide at 55°C for 18 hours, the oligonucleotides are purified by precipitation twice out of 0.5 M NaCl with 2.5 volumes ethanol. Analytical gel electrophoresis is accomplished in 20% acrylamide, 8 M urea, 45 mM Tris-borate buffer, pH 7.0.

### Screen for evaluating oligonucleotide functional groups for ability to inhibit capping of nascent mRNAs

The relative efficacy of various moieties and functional groups conjugated to oligonucleotides can be evaluated using the "G-less" transcript system of Sawadogo and Roeder [(1985) *Proc. Natl. Acad. Sci*. 82:4394-4398. While we do not wish to be limited to transcripts lacking guanosine residues, this assay allows screening of various moieties in a standardized manner. Those which are shown to be most active at preventing capping in this assay system are then incorporated into antisense oligonucleotides directed toward selected mRNA targets, such as ICAM-1 and CMV IE1/IE2. Briefly, this assay utilizes a HeLa cell extract for in vitro transcription of an exogenous DNA construct which encodes a G-less transcript. Such a transcript allows selective radiolabeling of capped mRNAs by the addition of [alpha-P32]-GTP, a substrate for guanylyltransferase. Only capped mRNAs will be radiolabeled.

### Example 2

### Inhibition of ICAM-1 mRNA capping

The sequence of the ICAM-1 mRNA transcript is known up to and including the 5'-most nucleotide. Degitz, K., Lian-Jie, L., and Caughman, S.W. (1991) *J. Biol. Chem.* 266:14024-14030. The 5' end of the nascent mRNA target is shown in Table 1, with 5'-triphosphate included:

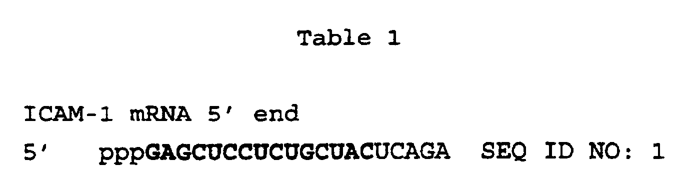

An antisense oligonucleotide complementary to the 5'-most nucleotides of the ICAM-1 sequence in Table 1 is synthesized according to Example 1. This oligonucleotide (SEQ ID NO:2) is shown in Table 2. A series of oligonucleotides are synthesized having SEQ ID NO: 2 and comprising an interfering moiety. These compounds are also shown in Table 2. Both phosphodiester (P=O) and phosphorothioate (P=S) oligonucleotides are shown.

The first such modified compound is an oligonucleotide which comprises two extra nucleotides on its 3' end such that they overhang the 5' end of the nascent ICAM-1 mRNA target SEQ ID NO: 3. This compound is readily prepared using the methods of automated oligonucleotide synthesis described in Example 1. In the example shown, the overhanging nucleotides are AG, but other nucleotides can also be used.

The second compound comprises an oligonucleotide having SEQ ID NO: 2 having a triethylene tetramine (Tren) moiety conjugated to the 3' end. This compound is prepared as follows: The oligonucleotide having SEQ ID NO: 2 is synthesized as in Example 1. Oxidation of the 3' ribose residue of the oligonucleotide is achieved with 20 mM sodium periodate in 100 mM sodium acetate (pH 5.2) for 4 hours at 4°C. Oligonucleotide concentration is 0.4 mM. The reaction is quenched with glycerol. The product is precipitated from solution with ethanol and dried under vacuum. Conjugation of the triethylene tetramine to the oxidized 3' ribose residue is performed by incubating 0.4 mM oxidized oligonucleotide, 20 mM Tren, 200 mM sodium bicarbonate (pH 9.2) for three hours at room temperature. The enamine conjugation product is reduced by addition of greater than ten equivalents (relative to oligonucleotide) of solid sodium borohydride directly to the conjugation reaction, mixing and incubating for one hour at 4°C. The reaction solution is purified by standard ion exchange chromatography using a Pharmacia FPLC system.

The third compound comprises an oligonucleotide having SEQ ID NO: 2 having an MPG [N-(2-mercaptopropionyl) glycine:Cu(II)] coordination complex conjugated to the 3' end. This compound is prepared via postmodification procedures. The oligonucleotide is functionalized during automated DNA synthesis with a conjugation group, usually a primary amine, sulfhydryl or ribose residue, at its 3' terminus. The MPG is derivatized with a complementary conjugation group, usually an aldehyde, carboxylic acid or primary amine. Sugiura, Y. and Hirayama, Y. (1976) *Inorg. Chem*.15:679-682; Sugiura, Y., Hirayama, Y., Tanaka, H. and Ishizu, K., (1975) *J. Am. Chem. Soc.* 97:5577-5581; Sugiura, Y., *Inorg. Chem*. 17:2176-2181. Conjugation is performed according to the postmodification procedures described by Uhlmann and Peyman [(1990) *Chem. Rev.* 90:543-584)].

The final compound shown comprises an oligonucleotide having SEQ ID NO: 2 with a tetra peptide lys-his-gly-his conjugated to the 3' end. This compound is prepared as follows: The oligonucleotide is synthesized as in Example 1. Oxidation of the 3' ribose residue of the oligonucleotide is achieved with 20 mM sodium periodate in 100 mM sodium acetate (pH 5.2) for 4 hours at 4°C. Oligonucleotide concentration is 0.4 mM. The reaction is quenched with glycerol. The product is precipitated from solution with ethanol and dried under vacuum. Conjugation of the peptide (N and C termini protected with FMOC and an amide group, respectively) via the ε-amino group of the lysine residue to the aldehyde groups of the oxidized 3' ribose residue is performed by incubating 0.4 mM oxidized oligonucleotide, 20 mM peptide, 200 mM sodium bicarbonate (pH 9.2) for three hours at room temperature. The enamine conjugation product is reduced by addition of greater than ten equivalents (relative to oligonucleotide) of solid sodium borohydride directly to the conjugation reaction, mixing and incubating for one hour at 4°C. The oligonucleotide-peptide conjugate is purified from the reducing reagent by size-exclusion chromatography (G10-120 Sephadex). The FMOC amino protecting group is removed in 1M triethylamine at room temperature in 30 minutes. The reaction solution is further purified by standard ion exchange chromatography using a Pharmacia FPLC system. Other peptides may generally be used as well.

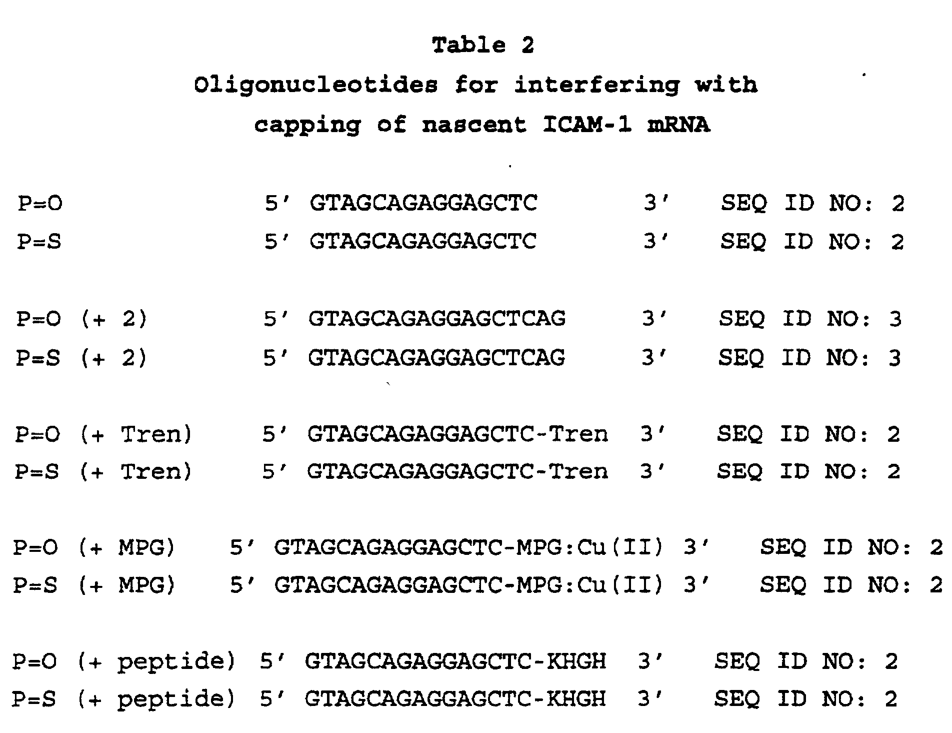

### Example 3

### Ability of compounds to block capping of ICAM-1 mRNA determined by fingerprinting assay for presence of 5' cap

A biochemical fingerprinting procedure for analysis of the 5' end of mRNA was originally used to define the chemical structure of the 5' cap [Cory, S. and Adams, J.M. (1975) *J. Mol. Biol*. 99:519-547]. This procedure is used here as an assay for the presence or absence of the methylated guanine cap structure. Briefly, the radiolabeled target mRNA is synthesized in vitro according to a generally used method such as that of Dignam et al. [(1983) Nucl. Acids. Res. 11:1475)] in the presence of oligonucleotide compounds. The RNA is purified and digested with a mixture of pancreatic, T1 and T2 ribonucleases and fractionated by electrophoresis on cellulose acetate at pH 3.5, followed by electrophoresis on DEAE paper at pH 3.5. The presence of an electrophoretic spot corresponding to 7-methyl guanosine 5'-phosphate indicates the presence of a 5' cap.

### Example 4

### Efficacy of compounds on blocking ICAM-1 expression in cell culture as determined by ELISA assay

The human lung carcinoma cell line A549 is obtained from the American Type Culture Collection (Bethesda MD). Cells are grown in Dulbecco's Modified Eagle's Medium (Irvine Scientific, Irvine CA) containing 1 mg glucose/liter and 10% fetal calf serum (Irvine Scientific). Human umbilical vein endothelial cells (HUVEC) (Clonetics, San Diego CA) are cultured in EGM-UV medium (Clonetics). HUVEC are used between the second and sixth passages. Human epidermal carcinoma A431 cells are obtained from the American Type Culture Collection and cultured in DMEM with 4.5 g/l glucose. Primary human keratinocytes are obtained from Clonetics and grown in KGM (Keratinocyte growth medium, Clonetics).

Cells grown in 96-well plates are washed three times with Opti-MEM (GIBCO, Grand Island, NY) prewarmed to 37°C. 100 µl of Opti-MEM containing either 10 µg/ml N-[1-(2,3-dioleyloxy)propyl]-N,N,N-trimethylammonium chloride (DOTMA, Bethesda Research Labs, Bethesda MD) in the case of HUVEC cells or 20 µg/ml DOTMA in the case of A549 cells is added to each well. Oligonucleotides are sterilized by centrifugation through 0.2 µm Centrex cellulose acetate filters (Schleicher and Schuell, Keene, NH). Oligonucleotides are added as 20x stock solution to the wells and incubated for 4 hours at 37°C. Medium is removed and replaced with 150 µl of the appropriate growth medium containing the indicated concentration of oligonucleotide. Cells are incubated for an additional 3 to 4 hours at 37°C then stimulated with the appropriate cytokine for 14 to 16 hours, as indicated. ICAM-1 expression is determined by ELISA assay: Cells are gently washed three times with a buffered isotonic solution containing calcium and magnesium such as Dulbecco's phosphate buffered saline (D-PBS). The cells are then directly fixed on the microtiter plate with 1 to 2% paraformaldehyde diluted in D-PBS for 20 minutes at 25°C. The cells are washed again with D-PBS three times. Nonspecific binding sites on the microtiter plate are blocked with 2% bovine serum albumin in D-PBS for 1 hour at 37°C. Cells are incubated with ICAM-1 monoclonal antibody diluted in blocking solution for 1 hour at 37°C. Unbound antibody is removed by washing the cells three times with D-PBS. Antibody bound to the cells is detected by incubation with a 1:1000 dilution of biotinylated goat anti-mouse IgG (Bethesda Research Laboratories, Gaithersberg, MD) in blocking solution for 1 hour at 37°C. Cells are washed three times with D-PBS and then incubated with a 1:1000 dilution of streptavidin conjugated to β-galactosidase (Bethesda Research Laboratories) for 1 hour at 37°C. The cells are washed three times with D-PBS for 5 minutes each. The amount of β-galactosidase bound to the ICAM-specific monoclonal antibody is determined by developing the plate in a solution of 3.3 mM chlorophenolred-β-D-galactopyranoside, 50 mM sodium phosphate, 1.5 mM MgCl₂; pH=7.2 for 2 to 15 minutes at 37°C. The concentration of the product is determined by measuring the absorbance at 575 nm in an ELISA microtiter plate reader.

### Example 5

### Inhibition of capping of cytomegalovirus IE1 and IE2 mRNA

The sequence of the CMV IE1/IE2 mRNA transcript is known. Stenberg, R.M., Witte, P.R. and Stinski, M.F. (1985) *J. Virol*. 56: 665-675; Stenberg, R.M., Thomsen, D.R., and Stinski, M.F. (1984) *J. Virol*. 49: 190-199. The 5' end of the nascent mRNA target shown in Table 3, with 5'-triphosphate included, is now known to terminate with a "G":

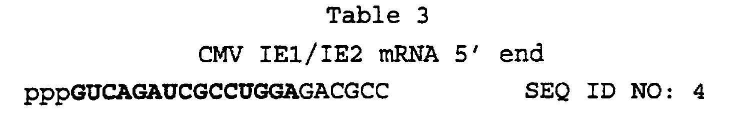

An antisense oligonucleotide complementary to the 5' -most nucleotides of the CMV IE1/IE2 sequence in Table 3 is synthesized according to Example 1. This oligonucleotide (SEQ ID NO:4) is shown in Table 4. A series of oligonucleotides are synthesized having SEQ ID NO: 4 and comprising an interfering moiety. These compounds are also shown in Table 4. Both phosphodiester (P=O) and phosphorothioate (P=S) oligonucleotides are shown.

The first such compound is an oligonucleotide which comprises two extra nucleotides on its 3' end such that they overhang the 5' end of the nascent CMV IE1/IE2 mRNA target. This compound is readily prepared using the methods of automated oligonucleotide synthesis described in Example 1. In the example shown, the overhanging nucleotides are AG, but other nucleotides can also be used.

The second compound comprises an oligonucleotide having SEQ ID NO:4 having a triethylene tetramine (Tren) moiety conjugated to the 3' end. This compound is prepared as follows: oxidation of the 3' ribose residue of the oligonucleotide is achieved with 20 mM sodium periodate in 100 mM sodium acetate (pH 5.2) for 4 hours at 4°C. Oligonucleotide concentration is 0.4 mM. The reaction is quenched with glycerol. The product is precipitated from solution with ethanol and dried under vacuum. Conjugation of the triethylene tetramine to the oxidized 3' ribose residue is performed by incubating 0.4 mM oxidized oligonucleotide, 20 mM Tren, 200 mM sodium bicarbonate (pH 9.2) for three hours at room temperature. The enamine conjugation product is reduced by addition of greater than ten equivalents (relative to oligonucleotide) of solid sodium borohydride directly to the conjugation reaction, mixing and incubating for one hour at 4°C. The reaction solution is purified by standard ion exchange chromatography using a Pharmacia FPLC system.

The third compound comprises an oligonucleotide having SEQ ID NO: 4 having an MPG [N-(2-mercaptopropionyl)glycine: Cu(II)] coordination complex conjugated to the 3' end. This compound is prepared via postmodification procedures. The oligonucleotide is functionalized during automated DNA synthesis with a conjugation group, usually a primary amine, sulfhydryl or ribose residue, at its 3' terminus. The MPG is derivatized with a complementary conjugation group, usually an aldehyde, carboxylic acid or primary amine. Sugiura, Y. and Hirayama, Y. (1976) *Inorg. Chem*.15:679-682; Sugiura, Y., Hirayama, Y., Tanaka, H. and Ishizu, K., (1975) *J. Am. Chem. Soc.* 97:5577-5581; Sugiura, Y., *Inorg. Chem.* 17:2176-2181. Conjugation is performed according to the postmodification procedures described by Uhlmann and Peyman [(1990) *Chem. Rev.* 90:543-584)].

The final compound shown comprises an oligonucleotide having SEQ ID NO:4 having a tetra peptide lys-his-gly-his conjugated to the 3' end. This compound is prepared as follows: oxidation of the 3' ribose residue of the oligonucleotide is achieved with 20 mM sodium periodate in 100 mM sodium acetate (pH 5.2) for 4 hours at 4°C. Oligonucleotide concentration is 0.4 mM. The reaction is quenched with glycerol. The product is precipitated from solution with ethanol and dried under vacuum. Conjugation of the peptide (N and C termini protected with FMOC and an amide group, respectively) via the ε-amino group of the lysine residue to the aldehyde groups of the oxidized 3' ribose residue is performed by incubating 0.4 mM oxidized oligonucleotide, 20 mM peptide, 200 mM sodium bicarbonate (pH 9.2) for three hours at room temperature. The enamine conjugation product is reduced by addition of greater than ten equivalents (relative to oligonucleotide) of solid sodium borohydride directly to the conjugation reaction, mixing and incubating for one hour at 4°C. The oligonucleotide-peptide conjugate is purified from the reducing reagent by size-exclusion chromatography (G10-120 Sephadex). The FMOC amino protecting group is removed in 1M triethylamine at room temperature in 30 minutes. The reaction solution is further purified by standard ion exchange chromatography using a Pharmacia FPLC system. Other peptides may also be used.

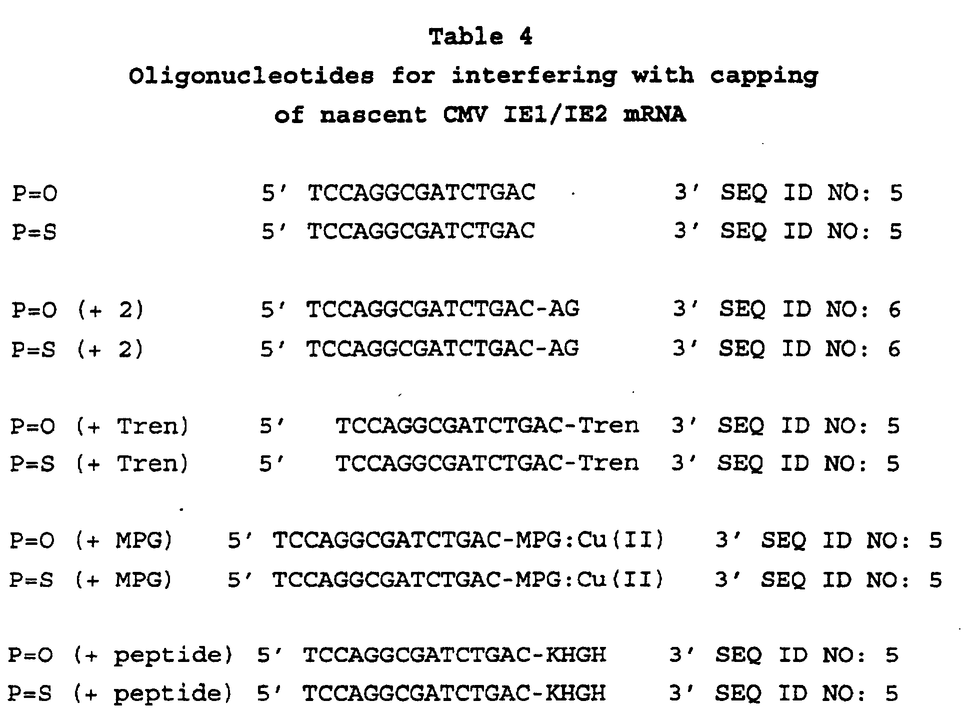

### Example 6

### Ability of compounds to block capping of ICAM-1 mRNA determined by fingerprinting assay for presence of 5' cap

A biochemical fingerprinting procedure for analysis of the 5' end of mRNA was originally used to define the chemical structure of the 5' cap [Cory, S. and Adams, J.M. (1975) *J*. *Mol. Biol.* 99:519-547]. This procedure is used here as an assay for the presence or absence of the methylated guanine cap structure. Briefly, the radiolabeled target mRNA is synthesized in vitro according to a generally used method such as that of Dignam et al. [(1983) Nucl. Acids. Res. 11:1475)] in the presence of oligonucleotide compounds. The RNA is purified and digested with a mixture of pancreatic, T1 and T2 ribonucleases and fractionated by electrophoresis on cellulose acetate at pH 3.5, followed by electrophoresis on DEAE paper at pH 3.5. The presence of an electrophoretic spot corresponding to 7-methyl guanosine 5'-phosphate indicates the presence of a 5' cap.

### Example 7

### ELISA assay for inhibition of CMV replication by oligonucleotides which block capping of IE1/IE2 mRNA

Oligonucleotides shown in Table 4 are tested for antiviral activity in an ELISA-based assay of CMV replication. Normal human dermal fibroblasts (Clonetics Corp., San Diego CA) are grown in serum-free medium (Clonetics) and used to seed 96-well plates. When cells are approximately 80% confluent, they are pretreated with compounds. Approximately 20 hours after pretreatment the medium (containing oligonucleotide) is carefully poured off and the cells washed twice with warmed fibroblast basal medium (FBM, Clonetics). Cells are then infected with 100 µl/well of CMV stock diluted in FBM. The plates are incubated at 37°C for two hours. The medium (containing virus) is then carefully poured off and replaced with fresh, prewarmed FBM medium, 100µl per well. The plates are briefly incubated at 37°C and then 5 µl of compound, diluted in FBM, is reintroduced into the medium in each well. Two days later, cells are post-treated again with oligonucleotides in the same way. On day six, the plates are prepared for ELISA.

In preparation for ELISA, the medium is carefully poured off the plates, and cells are fixed in 200 µl of absolute ethanol per well. Cells are fixed for 30 minutes at room temperature, then ethanol is removed and plates are air-dried. Plates are blocked for one hour prior to ELISA with PBS containing 2% BSA. Blocking solution is removed and 100 µl of an anti-CMV antibody, diluted 1:2000 in PBS with 1% BSA, is added. Cells are incubated in antibody for one hour at 37°C and washed three times in PBS. The secondary antibody, biotinylated goat anti-mouse IgG (Bethesda Research Labs, MD), is diluted 1:1000 in PBS with 1% BSA, and incubated with cells for one hour at 37°C. Cells are then washed and incubated for one hour at 37°C in streptavidin-B-D-galactosidase. Color is developed with chlorophenol red-B-D-galactopyranoside, 20 mg dissolved in 10 ml of 50 mM Na Phosphate, 1.5 mM MgCl2; plates are shaken for 10 minutes and the absorbance is read at 575 nm.

## Claims

1. Use of an antisense oligonucleotide for inhibiting the capping of a nascent mRNA molecule.

2. Use according to claim 1, wherein said antisense oligonucleotide is specifically hybridizable with said nascent mRNA molecule.

3. Use according to claim 2, wherein said oligonucleotide is specifically hybridizable with a 5' region of said nascent mRNA molecule.

4. Use according to any one of claims 1 to 3, wherein said oligonucleotide is 10 to 25 nucleotides in length.

5. Use according to any one of the preceding claims, wherein the nascent mRNA molecule is from 20 to 80 nucleotides in length.

6. Use according to any one of the preceding claims, wherein said oligonucleotide is capable of sterically interfering with capping of the nascent mRNA molecule.

7. Use according to claim 6, wherein said oligonucleotide comprises at the 3' position at least one RNA capping interfering moiety.

8. Use according to claim 7, wherein said interfering moiety comprises at least one amino acid, polyamine, co-ordination complex of a metal, metal ion or macrocycle.

9. Use according to claim 8, wherein said polyamine is triethylamine tetramine, diethylene triamine or pentethylamine hexamine.

10. Use according to claim 8, wherein said co-ordination complex of a metal is copper, zinc, cobalt or lanthanide complex of bipyridine, orthophenanthroline, 12aneN3, diethylenetriamine pentaacetic acid, or N-[2-mercaptopropionyl] glycine.

11. Use according to claim 8, wherein said metal ion is cobalt, zinc, copper, europium, gadolinium, calcium or magnesium.

12. Use according to claim 8, wherein said macrocycle is [24]ane N₆O₂.

13. Use according to any one of claims 1-7, wherein said oligonucleotide comprises at least one nucleotide at the 3' end which extends beyond the nucleotide units of the 5' portion of the nascent mRNA.

14. Use according to any one of the preceding claims, wherein said oligonucleotide is capable of chemically modifying the nascent mRNA.

15. Use according to claim 14, wherein the chemical modification is dephosphorylation of the 5' - triphosphate or 5' - diphosphate of the nascent mRNA.

16. Use according to claim 15, wherein the dephosphorylation yields a 5' - monophosphate or a hydroxyl group.

17. Use according to any one of the preceding claims, wherein the capping enzyme is a guanylyltransferase.

18. An antisense oligonucleotide 10 to 25 nucleotides in length, specifically hybridizable with a 5' region of preselected nascent mRNA, and comprising at the 3' position of the oligonucleotide at least one RNA capping interfering moiety, wherein said interfering moiety comprises an amino acid; tetrapeptide lys-his-gly-his; pentethylamine hexamine; copper or zinc complex of 12aneN3, diethylenetriamine pentaacetic acid, or N-[2-mercaptopropionyl] glycine; cobalt or lanthanide complex of bipyridine, orthophenanthroline, 12aneN3, diethylenetriamine pentaacetic acid, or N-[2-mercaptopropionyl] glycine; transition metals cobalt, zinc, or copper; lanthanide metals europium or gadolinium; divalent cations calcium or magnesium; or a macrocycle.

19. An antisense oligonucleotide according to claim 18, wherein said interfering moiety is an amino acid, tetrapeptide lys-his-gly-his, or a macrocycle.

20. An antisense oligonucleotide according to claim 18 or claim 19, for use as a medicament.

## Patentansprüche

1. Verwendung eines Antlsense-Oligonukleotids zum Inhibieren der Cap-Bildung eines naszierenden mRNA-Moleküls.

2. Verwendung nach Anspruch 1, wobei das Antisense-Oiigonukieotid mit dem naszierenden mRNA-Molekül spezifisch hybridisierbar ist.

3. Verwendung nach Anspruch 2, wobei das Oligonukleotid mit einem 5'-Bereich des naszierenden mRNA-Moleküls spezifisch hybridisierbar ist.

4. Verwendung nach einem der Ansprüche 1 bis 3, wobei das Oligonukleotid 10 - 25 Nukleotide lang ist.

5. Verwendung nach einem der vorhergehenden Ansprüche, wobei das naszierende mRNA-Molekül 20 - 80 Nukleotide lang ist.

6. Verwendung nach einem der vorhergehenden Ansprüche, wobei das Oligonukleotid befähigt ist, sterisch die Cap-Bildung des naszierenden mRNA-Moleküls zu beeinflussen.

7. Verwendung nach Anspruch 6, wobei das Oligonukleotid an der 3'-Position mindestens einen die RNA-Cap-Bildung beinflussenden Teil umfaßt.

8. Verwendung nach Anspruch 7, wobei der beeinflussende Teil mindestens eine Aminosäure, ein Polyamin, einen Koordinationskomplex eines Metalls, ein Metallion oder einen Makrozyklus umfaßt.

9. Verwendung nach Anspruch 8, wobei das Polyamin Triethylamintetramin, Diethylentriamin oder Pentethylaminhexamin ist.

10. Verwendung nach Anspruch 8, wobei der Koordinationskomplex eines Metalls ein Kupfer-, Zink-, Kobalt- oder Lanthanoidkomplex von Bipyridin, ortho-Phenanthrolin, 12aneN3, Diethylentriaminpentaessigsäure oder N-[2-Mercaptopropionyl]glycin ist.

11. Verwendung nach Anspruch 8, wobei das Metallion Kobalt, Zink, Kupfer, Europium, Gadolinium, Calcium oder Magnesium ist.

12. Verwendung nach Anspruch 8, wobei der Makrozyklus [24]aneN₆O₂ ist.

13. Verwendung nach einem der Ansprüche 1 bis 7, wobei das Oligonukleotid mindestens ein Nukleotid am 3'-Ende umfaßt, welches sich über die Nukleotideinheiten des 5'-Bereichs der naszierenden mRNA erstreckt.

14. Verwendung nach einem der vorhergehenden Ansprüche, wobei das Oligonukleotid befähigt ist, die naszierende mRNA chemisch zu modifizieren.

15. Verwendung nach Anspruch 14, wobei die chemische Modifikation eine Dephosphorylierung des 5'-Triphosphats oder 5'-Diphosphats der naszierenden mRNA ist.

16. Verwendung nach Anspruch 15, wobei die Dephosphorylierung ein 5'-Monophosphat oder eine Hydroxylgruppe ergibt.

17. Verwendung nach einem der vorhergehenden Ansprüche, wobei das Capbildende Enzym eine Guanylyltransferase ist.

18. Antisense-Oligonukleotid mit 10 bis 25 Nukleotiden in der Länge, spezifisch hybridisierbar mit einem 5'- Bereich einer vorausgewählten naszierenden mRNA und umfassend an der 3'-Position des Oligonukleotids mindestens einen die RNA-Cap-Bildung beinflussenden Teil, wobei der beinflussende Teil eine Aminosäure; ein Tetrapeptid Lys-His-Gly-His; Pentethylaminhexamin; einen Kupfer- oder Zinkkomplex von 12aneN3, Diethylentriaminpentaessigsäure oder N-[2-Mercaptopropionyl]glycin; einen Kobalt- oder Lanthanoidkomplex von Bipyridin, ortho-Phenanthrolin, 12aneN3, Diethylentriaminpentaessigsäure oder N-[2-Mercaptopropionyl]glycin; Übergangsmetalle Kobalt, Zink oder Kupfer; Lanthanoidmetalle Europium oder Gadolinium; zweiwertige Kationen Calcium oder Magnesium oder einen Makrozyklus umfaßt.

19. Antisense-Oligonukleotid nach Anspruch 18, wobei der beinflussende Teil eine Aminosäure, ein Tetrapeptid Lys-His-Gly-His oder ein Makrozyklus ist.

20. Antlsense-Oligonukleotid nach Anspruch 18 oder 19 zur Verwendung als ein Medikament.

## Revendications

1. Utilisation d'un oligonucléotide anti-sens pour inhiber le coiffage d'une molécule d'ARNm naissante.

2. Utilisation selon la revendication 1, dans laquelle ledit oligonucléotide anti-sens peut s'hybrider spécifiquement à ladite molécule d'ARNm naissante.

3. Utilisation selon la revendication 2, dans laquelle ledit oligonucléotide peut s'hybrider spécifiquement avec une région 5' de ladite molécule d'ARNm naissante.

4. Utilisation selon l'une quelconque des revendications 1 à 3, dans laquelle ledit oligonucléotide a une longueur de 10 à 25 nucléotides.

5. Utilisation selon l'une quelconque des revendications précédentes, dans laquelle la molécule d'ARNm naissante a une longueur de 20 à 80 nucléotides.

6. Utilisation selon l'une quelconque des revendications précédentes, dans laquelle ledit oligonucléotide est capable d'interférer de façon stérique avec le coiffage de la molécule d'ARNm naissante.

7. Utilisation selon la revendication 6, dans laquelle ledit oligonucléotide contient à la position 3' au moins un fragment interférant dans le coiffage de l'ARN.

8. Utilisation selon la revendication 7, dans laquelle ledit fragment interférant contient au moins un acide aminé, une polyamine, un complexe de coordination d'un métal, d'ion métallique ou de macrocycle.

9. Utilisation selon la revendication 8, dans laquelle ladite polyamine est la triéthylamine tétramine, la diéthylamine triamine ou la pentéthylamine hexamine.

10. Utilisation selon la revendication 8, dans laquelle ledit complexe de coordination d'un métal est un complexe de cuivre, de zinc, de cobalt ou de lanthanide de bipyridine, d'orthophénanthroline, de 12aneN3, d'acide pentaacétique de diéthylènetriamine ou de N-[2-mercaptopropionyl]-glycine.

11. Utilisation selon la revendication 8, dans laquelle ledit ion métallique est le cobalt, le zinc, le cuivre, l'europium, le gadolinium, le calcium ou le magnésium.

12. Utilisation selon la revendication 8, dans laquelle le dit macrocycle est le [24]aneN₆O₂.

13. Utilisation selon l'une quelconque des revendications 1 à 7, dans laquelle ledit oligonucléotide contient au moins un nucléotide à l'extrémité 3' qui s'étend au-delà des unités de nucléotides de la portion 5' de l'ARNm naissant.

14. Utilisation selon l'une quelconque des revendications précédentes, dans laquelle ledit oligonucléotide est capable de modifier chimiquement l'ARNm naissant.

15. Utilisation selon la revendication 14, dans laquelle ladite modification chimique est une déphosphorylation du 5'-triphosphate ou 5'-diphosphate de l'ARNm naissant.

16. Utilisation selon la revendication 15, dans laquelle la déphosphorylation donne un 5'-monophosphate ou un groupement hydroxyle.

17. Utilisation selon l'une quelconque des revendications précédentes, dans laquelle l'enzyme de coiffage est la guanylyl-transférase.

18. Oligonucléotide anti-sens d'une longueur de 10 à 25 nucléotides, capable de s'hybrider spécifiquement avec une région 5' d'un ARNm naissant prsélectionné, et comprenant en position 3' de l'oligonucléotide au moins un fragment interférant de coiffage d'ARN, dans lequel ledit fragment interférant contient un acide aminé ; le tétrapeptide lys-his-gly-his ; la pentéthylamine hexamine ; un complexe cuivre ou zinc de 12aneN3, d'acide diéthylènetriamine pentaacétique, ou le N-[2-mercaptopropionyl] glycine ; un complexe de cobalt ou de lanthanide de bipyridine, d'orthophénanthroline, de 12aneN3, d'acide diéthylènetriamine pentaacétique ou de N-[2-mercaptopropionyl] glycine; des métaux de transition cobalt, zinc ou cuivre; des métaux lanthanides europium ou gadolinium ; des cations divalents calcium ou magnésium ou un macrocycle.

19. Oligonucléotide anti-sens selon la revendication 18, dans lequel ledit fragment interférant est un acide aminé, le tétrapeptide lys-his-gly-his ou un macrocycle.

20. Oligonucléotide anti-sens selon la revendication 18 ou la revendication 19, pour une utilisation en tant que médicament.


### SEQUENCE LISTING

(1) GENERAL INFORMATION:
   (i) APPLICANT: ISIS Pharmaceuticals and Brenda Baker
   (ii) TITLE OF INVENTION: Compositions and Methods for Modulating
      RNA Activity Through Interference with Capping of Nascent RNA
   (iii) NUMBER OF SEQUENCES: 6
   (iv) CORRESPONDENCE ADDRESS:
      (A) ADDRESSEE: Woodcock Washburn Kurtz Mackiewicz & Norris
      (B) STREET: One Liberty Place - 46th Floor
      (C) CITY: Philadelphia
      (D) STATE: PA
      (E) COUNTRY: USA
      (F) ZIP: 19103
   (v) COMPUTER READABLE FORM:
      (A) MEDIUM TYPE: DISKETTE, 3.5 INCH, 1.44 Mb STORAGE
      (B) COMPUTER: IBM PS/2
      (C) OPERATING SYSTEM: PC-DOS
      (D) SOFTWARE: WORDPERFECT 5.0
   (vi) CURRENT APPLICATION DATA:
      (A) APPLICATION NUMBER:n/a
      (B) FILING DATE: herewith
      (C) CLASSIFICATION:
   (vii) PRIOR APPLICATION DATA:
      (A) APPLICATION NUMBER: US 847,054
      (B) FILING DATE: 03-APR-1992
   (vii) PRIOR APPLICATION DATA:
      (A) APPLICATION NUMBER: PCT/US91/03636
      (B) FILING DATE: 22-MAY-1991
   (vii) PRIOR APPLICATION DATA:
      (A) APPLICATION NUMBER: 527,559
      (B) FILING DATE: 23-MAY-1990
   (viii) ATTORNEY/AGENT INFORMATION:
      (A) NAME: John W. Caldwell
      (B) REGISTRATION NUMBER: 28,937
      (C) REFERENCE/DOCKET NUMBER: ISIS-1120
   (ix) TELECOMMUNICATION INFORMATION:
      (A) TELEPHONE: (215) 568-3100
      (B) TELEFAX: (215) 568-3439
(2) INFORMATION FOR SEQ ID NO: 1:
   (i) SEQUENCE CHARACTERISTICS:
      (A) LENGTH: 20
      (B) TYPE: nucleic acid
      (C) STRANDEDNESS: single
      (D) TOPOLOGY: unknown
   (iv) ANTISENSE: no
   (xi) SEQUENCE DESCRIPTION: SEQ ID NO: 1:
   GAGCUCCUCU GCUACUCAGA (20)
(2) INFORMATION FOR SEQ ID NO: 2:
   (i) SEQUENCE CHARACTERISTICS:
      (A) LENGTH: 15
      (B) TYPE: nucleic acid
      (C) STRANDEDNESS: single
      (D) TOPOLOGY: unknown
   (iv) ANTISENSE: yes
   (xi) SEQUENCE DESCRIPTION: SEQ ID NO: 2:
   GTAGCAGAGG AGCTC (15)
(2) INFORMATION FOR SEQ ID NO: 3:
   (i) SEQUENCE CHARACTERISTICS:
      (A) LENGTH: 17
      (B) TYPE: nucleic acid
      (C) STRANDEDNESS: single
      (D) TOPOLOGY: unknown
   (iv) ANTISENSE: yes
   (xi) SEQUENCE DESCRIPTION: SEQ ID NO: 3:
   GTAGCAGAGG AGCTCAG (17)
(2) INFORMATION FOR SEQ ID NO: 4:
   (i) SEQUENCE CHARACTERISTICS:
      (A) LENGTH: 21
      (B) TYPE: nucleic acid
      (C) STRANDEDNESS: single
      (D) TOPOLOGY: unknown
   (iv) ANTISENSE: no
   (xi) SEQUENCE DESCRIPTION: SEQ ID NO: 4:
   GUCAGAUCGC CUGGAGACGC C (21)
(2) INFORMATION FOR SEQ ID NO: 5:
   (i) SEQUENCE CHARACTERISTICS:
      (A) LENGTH: 15
      (B) TYPE: nucleic acid
      (C) STRANDEDNESS: single
      (D) TOPOLOGY: unknown
   (iv) ANTISENSE: yes
   (xi) SEQUENCE DESCRIPTION: SEQ ID NO: 5:
   TCCAGGCGAT CTGAC (15)
(2) INFORMATION FOR SEQ ID NO: 6:
   (i) SEQUENCE CHARACTERISTICS:
      (A) LENGTH: 17
      (B) TYPE: nucleic acid
      (C) STRANDEDNESS: single
      (D) TOPOLOGY: unknown
   (iv) ANTISENSE: yes
   (xi) SEQUENCE DESCRIPTION: SEQ ID NO: 6:
   TCCAGGCGAT CTGACAG (17)